(19) Europäisches Patentamt
European Patent Office
Office européen des brevets

(11) **EP 2 721 999 A1**

(12) **EUROPÄISCHE PATENTANMELDUNG**

(43) Veröffentlichungstag:
**23.04.2014  Patentblatt 2014/17**

(51) Int Cl.:
*A61B 5/024* <sup>(2006.01)</sup>     *A61B 5/0402* <sup>(2006.01)</sup>

(21) Anmeldenummer: **12188701.2**

(22) Anmeldetag: **16.10.2012**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder:
• **xotoxtools ag
8700 Küsnacht (CH)**
• **EMPA Eidgenössische Materialprüfungs- und
Forschungsanstalt
CH-8600 Dübendorf (CH)**

(72) Erfinder:
• **Bachmann, Philipp
8700 Kuesnacht (CH)**
• **Fritzsche, Rolf
8340 Hinwil (CH)**

(74) Vertreter: **Schmauder & Partner AG
Patent- & Markenanwälte VSP
Zwängiweg 7
8038 Zürich (CH)**

(54) **Vorrichtung und Verfahren zum Erkennen und Melden eines Belastungszustandes einer Person**

(57)     Um den momentanen Stresszustand einer Person einfach bestimmen zu können wird eine Vorrichtung vorgeschlagen, die die Pulsrate misst und daraus zusätzlich die Herzratenvariabilität bestimmt. Zusätzlich soll mindestens ein Parameter zur Historie einer der beiden vorgenannten Werte verwendet werden. Vorzugsweise wird die Abweichung der Pulsrate und der Herzratenvariabilität von einer Normgrösse integriert und als zusätzlicher Stressindikator verwendet.

EP 2 721 999 A1

**Beschreibung**

Technisches Gebiet

Die Erfindung betrifft eine Vorrichtung und ein Verfahren zum Erkennen und Melden eines Belastungszustandes einer Person.

Stand der Technik

Für die Ermittlung von Stresszuständen die Pulsrate oder die Herzratenvariabilität zu verwenden, ist seid langem bekannt. Dabei wird der Abstand zwischen zwei Herzschlägen - im Sinne dieser Erfindung - definiert als die Zeit zwischen dem Beginn zweier Kontraktionen der Herzkammern. Dieser Beginn der Kammerkontraktion wird im Elektrokardiogramm (EKG) als R-Zacke dargestellt. Der Abstand zwischen zwei R-Zacken wird üblicherweise als RR-Intervall bezeichnet. Nach einer Mittelung über eine bestimmte Anzahl von RR-Intervallen kann man rechnerisch die Herzfrequenz bestimmen. Die einzelnen Werte der RR-Intervalle schwanken um den so erhaltenen Mittelwert. Dabei können sich die Abweichungen von Schlag zu Schlag ändern. Die Abweichung wird zumeist als Herzratenvariabilität (HRV) bezeichnet. Grundsätzlich kann die Herzfrequenz auch durch eine Druckmessung an einer Arterie vorgenommen werden.

Physiologisch hängt die Herzratenvariabilität (HRV) mit der Fähigkeit des menschlichen Organismus zusammen, die Frequenz des Herzrhythmus anzupassen. Herzratenänderungen, also Veränderungen des zeitlichen Abstandes zwischen zwei Herzschlägen, treten sowohl im Ruhezustand, dann zumeist spontan, wie auch bei bestimmten Veränderungen des Umfeldes, z.B. Belastungen auf. Über physiologische Regulationswege des vegetativen Nervensystems passt ein gesunder Organismus die Herzschlagrate beständig momentanen Erfordernissen an. Körperliche Beanspruchung oder psychische Belastung hat deswegen in der Regel eine Erhöhung der Herzfrequenz zur Folge, die bei Entlastung und Entspannung normalerweise wieder zurückgeht. Dabei zeigt sich eine gute Anpassungsfähigkeit an Belastungen in einer höheren Variabilität der Herzfrequenz. Unter chronischer Stressbelastung ist die Anpassungsfähigkeit reduziert. Insofern bietet - dies ist bekannt - die Herzratenvariabilität für sich genommen schon einen gewissen, allerdings noch sehr unzuverlässigen Indikator für die momentane Stressbelastungen sowie die Stressbelastungsfähigkeit einer Person.

Im Stand der Technik wurden vielfach Verfahren zur Bestimmung des Belastungszustandes (Stresszustandes) einer Person vorgeschlagen, wobei vorgeschlagen wurde, neben der Pulsrate weitere Messgrössen zu verwenden. So wird in der DE 103 19 361 A1 vorgeschlagen, neben der Herzratenvariabilität die Pulswellenlatenz zu verwenden.

In Bezug auf die Auswertung der Herzratenvariabilität wird auf die DE 100 06 154 A1, die DE 10 2006 039 957 A1, sowie die DE 10 2008 030 956 A1 und die EP 1 156 851 B1 verwiesen, in denen der Fachmann verschiedene Verfahren zur Bestimmung finden kann.

Aus der EP-2 136 333 A1 ist eine Vorrichtung und eine Verfahren bekannt, bei der eine Zustandsgrösse berechnet wird, die eine Funktion der momentanen Pulsfrequenz und der momentanen Herzratenvariabilität ist, vorzugsweise eine Linearkombination der momentanen Pulsfrequenz P und der momentanen Herzratenvariabilität ist. Weiterhin ist in der genannten Druckschrift vorgeschlagen worden, die Zustandsfunktion mit zumindest einer Korrekturgrösse zu versehen, die die Vorgeschichte der Person zumindest innerhalb der letzten 0,5 Stunden berücksichtigt.

Dieser Ansatz erscheint dabei durchaus geeignet. Es ergibt sich aber ein weiteres Optimierungsproblem, welche dadurch beschrieben werden kann, dass einerseits - insbesondere zum Vergleich verschiedener Personen - Normierungen eingeführt werden müssen, aber andererseits auch individuelle Unterschiede berücksichtigt werden sollen.

Darstellung der Erfindung

Aufgabe der Erfindung ist es, eine Vorrichtung und ein entsprechendes Verfahren zum Erkennen und Melden eines Belastungszustandes einer Person mit einer - gegenüber dem Stand der Technik - erhöhten Zuverlässigkeit zur Verfügung zu stellen und bei denen einerseits - insbesondere zum Vergleich verschiedener Personen - Normierungen eingeführt werden können, aber andererseits auch individuelle Unterschiede berücksichtigt werden.

Die Aufgabe der Erfindung wird - gemäss einem ersten Aspekt - durch ein Verfahren nach Anspruch 1 gelöst. Die Massnahmen der Erfindung haben zunächst einmal zur Folge, dass in einer ersten Näherung, nämlich im ersten Verarbeitungsfenster, also in einem ersten Zeitintervall $T_1$ oder einer vorgegebenen Anzahl von Pulsschlägen, ein erstes Näherungsergebnis erzielt wird, mit welchem bereits gearbeitet werden kann. Hierzu bieten sich insbesondere die Alterswerte an, die - vorzugsweise in jeweiligen Altersgruppen - typische Tabellenwerte für die Minimal- und die Maximalwerte der Pulsrate und des HRV-Wertes liefern. Die weiteren Verarbeitungsfenster können dann - gemäss experimentellen Studien - Ergebnisse liefern, die insbesondere dann verbessert sind, wenn die Person in ihrer Charakteristik von den Tabellenwerten für die Minimal- und die Maximalwerte der Pulsrate und des HRV-Wertes abweichen, sei es aus Gründen z.B. des besonders guten oder besonders schlechten Trainingsstandes oder auch wegen hoher oder niedriger Stresswerte in der näheren oder mittleren Historie. Es sollte bemerkt werden, dass die Pulsaufnahme irgendwo am Körper möglich ist, wobei man grundsätzlich optisch, akustisch oder kinästhetisch messen und in elektrische Signale umwandeln kann. Auch eine direkte Messung elektrischer Signale zur Pulsmessung ist für die Erfindung möglich.

Entsprechend dem Verfahren zum Erkennen und Melden eines Belastungszustandes einer Person gemäss der vorliegenden Erfindung werden dabei folgende Schritte durchgeführt:

Es werden in dem Zeitintervall oder für eine vorgegebene Zahl von Pulsschlägen fortlaufend die Daten der momentanen Pulsfrequenz P und der momentanen Herzratenvariabilität HRV erfasst und verarbeitet. Dabei wird getrennt für die Pulsrate und die Herzratenvariabilität ein Wert für den Stressindex berechnet und anschliessend die beiden Werte - mit Gewichtungsfaktoren - addiert. Die Werte der Pulsraten und der Herzratenvariabilität werden dabei mit Hilfe von - vorzugsweise altermässig sortierten - tabellenmässig erwarteten Extremwerten $P_{max}$, $P_{min}$, $HRV_{max}$ und $HRV_{min}$ normiert. Damit wird ein erster- möglicherweise schon brauchbarer Wert - für den Stressindex ermittelt.

Weiter wird aber für das Zeitintervall oder die vorbestimmte Zahl von Pulsschlägen ermittelt, ob die persönlichen Extremwerte $P_{max}$, $P_{min}$, $HRV_{max}$ und $HRV_{min}$ anders sind als die zuvor verwendeten Werte. Für das nächste Zeitintervall oder für die nächste Messung von vorgegebenen Pulsschlägen, wobei weder die Länge des Zeitintervalls noch die Zahl von Pulsschlägen notwendigerweise identisch sein müssen wie für die erste Messung, und welches auch deutlich später, also mit einem zeitlichen Zwischenraum von bis zu mehreren Stunden liegen kann, werden dann entweder die alten Werte oder die neu ermittelten Extremwerte verwendet, je nachdem, welche Werte extremer sind. Die Berechnung des neuen Wertes für den Stressindex geschieht dann prinzipiell ebenso wie die beschriebene erste Messung, also fortlaufendes getrenntes Ermitteln der Stressindexwerte für die Pulsrate und die Herzratenvariabilität ein Wert für den Stressindex berechnet und anschliessendes addieren der beiden Werte mit Gewichtungsfaktoren. Das Zeitintervall wird dabei typischerweise im Bereich von 100s bis 1000 s - vorzugsweise 300s bis 500s - liegen, kann aber auch jeweils um eine Grössenordnung (Faktor 10) kleiner oder grösser sein. Die Zahl der Pulsschläge wird zwischen 50 und 500, vorzugsweise 100 liegen, kann aber insbesondere auch um ein bis zwei Grössenordnungen grösser sein. Es sollte betont werden, dass die Folgemessungen sich durchaus auch zeitlich überlappen können, wobei dann bei Beginn der neuen Messreihe selbstverständlich nur die Extremwerte verwendet werden können, die zuvor aufgetreten sind. Diese "Movingwindow" Methode kann dann sinnvoll sein, wenn sehr viel Rechenkapazität zur Verfügung steht und ein sehr schnelles Ergebnis erzielt werden soll.

Vorteilhaft ist das Verfahren durchführbar, wenn die Normierung jeweils mittels eines Normierungswertes bei der ersten, tabellenbezogenen

$$P_z = P_{min} + a*(P_{max} - P_{min})$$

$$HRV_z = HRV_{min} + b* (HRV_{max} - HRV_{min})$$

und die Berechnung der Summanden für des Stresswert SI jeweils mittels

$$SI_P = (P_{d1} - P_z)/(P_{max} - P_z) \text{ wenn } P_{d1} > P_z$$

$$SI_P = (P_{d1} - P_z)/(P_z - P_{min}) \text{ wenn } P_{d1} < P_z$$

$$SI_{HRV} = -(HRV_{d1} - HRV_z)/(HRV_{max} - HRV_z) \text{ wenn } HRV_{d1} > HRV_z$$

$$SI_{HRV} = -(HRV_{d1} - HRV_z)/(HRV_z - HRV_{min}) \text{ wenn } HRV_{d1} < HRV_z$$

erfolgt.
Die weiteren Fenster werden dann vorteilhafterweise berechnet gemäss

$$SI_P=(P_{dx}-P_z)/(P_{max}-P_z) \text{ wenn } P_{dx} > P_z$$

$$SI_P=(P_{dx}-P_z)/(P_z-P_{min}) \text{ wenn } P_{dx} < P_z$$

$$SI_{HRV}=-(HRV_{dx}-HRV_z)/(HRV_{max}-HRV_z) \text{ wenn } HRV_{dx} > HRV_z$$

$$SI_{HRV}=-(HRV_{dx}-HRV_z)/(HRV_z-HRV_{min}) \text{ wenn } HRV_{dx} < HRV_z.$$

Vorteilhaft hat sich herausgestellt, wenn die Werte a für die Verarbeitung der Pulsrate gewählt wird im Bereich zwischen 0.2 und 0.3, vorteilhafterweise als 0.25 und die Werte b für die Verarbeitung der Herzratenvariabilität zwischen 0.33 bis 0.66, vorteilhafterweise als 0.5.

Das Verfahren ist - je nach Anwendungsfall - durchaus kalibrierbar in dem Sinne, dass die Gewichtung des Stressindexteilwertes, der aus der Pulsrate ermittelt wird und dem Stressindexteilwert, der aus der Herzratenvariabilität ermittelt wird, durch Tests optimiert wird. Sofern dazu keine Veranlassung besteht, wird man die Werte c und d wählen als 1.

Besonders vorteilhaft ist es, wenn die jeweiligen ermittelten Stresswerte nicht nur direkt ausgegeben werden, was aber selbstverständlich nicht ausgeschlossen ist, sondern der aktuelle Stressindex SI nach dem Zeitintervall $T_x$ oder nach der vorgegebenen Anzahl von Pulsschlägen einem Filter, typischerweise einem digitalen Tiefpass, zugeführt wird gewählt wird, um einzelne Ausreisser mitteln zu können, z.B. $SI = f*SI_x+(1-f)*SI_{x-1}$, mit f zwischen 0.05 und 0.5, vorteilhafterweise von 0.1.

Gemäss einem zweiten Aspekt der vorliegenden Erfindung wird eine Vorrichtung vorgeschlagen, die zur Durchführung des erfindungsgemässen Verfahrens, einschliesslich der vorstehend genannten vorteilshaften Ausführungsformen, möglicherweise aber auch ohne diese, geeignet ist.

Eine solche Vorrichtung zum Erkennen und Melden eines Belastungszustandes einer Person, wird typischer Weise eine Erfassungseinrichtung zum fortlaufenden Erfassen der Daten der momentanen Pulsfrequenz und der momentanen Herzratenvariabilität, eine Verarbeitungseinrichtung zum fortlaufenden Verarbeiten der Daten der momentanen Pulsfrequenz und der momentanen Herzratenvariabilität, und eine Vergleichseinrichtung zum Vergleichen der gewonnenen momentanen Zustandsfunktion der Person mit einem Alarmkriterium umfassen.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können. Die Berechnung der Herzratenvariabilität kann zur Durchführung des erfindungsgemässen Verfahrens durch übliche Methoden (z.B. in der Zeitdomäne: RMSSD, RRinter, SDNN, oder in der Frequenzdomäne $LF_{tot}/HF_{tot}$) durchgeführt werden, wobei die Methode RMSSD ("Root mean square of sucessive differences") für die hier genannten Methode und Vorrichtung geeignet ist. Es solle darauf hingewiesen werden, dass das erfinderische Verfahren nicht dazu gedacht, den Gesundheits- oder Krankheitszustand der Person festzustellen, während die erfindungsgemässe Vorrichtung diesbezüglich keiner Einschränkung unterliegt.

Wege zur Ausführung der Erfindung

Die erfindungsgemässe Vorrichtung umfasst gemäss einem bevorzugten Ausführungsbeispiel der Erfindung eine Messeinrichtung zur Ermittlung der Pulsrate und der Werte, die zur Berechnung einer Herzratenvariabilität notwendig sind. Dies ist hier ein Pulsmesssensor, kann aber auch alternativ ein elektrischer Sensor zur Messung von elektrischen kardiografischen Messwerten sein, sowie ein Anzeigegerät. Weiterhin umfasst die Vorrichtung eine Schnittstelle zur Eingabe von personenbezogenen Grössen, die insbesondere zur Ermittlung der erfindungsgemäss zu verwendenden Historie notwendig sind. Kernstück der Vorrichtung ist eine Recheneinrichtung, die die notwendige Messdatenerfassung kontrolliert, die Messdatenaufbereitung in der notwendigen digitalen Form durchführt, die Datenverarbeitung durchführt und die Anzeige bedient.

Im vorliegenden Ausführungsbeispiel wird die Herzratenvariablilität HRV bestimmt durch die RMSSD-Methode ("Root mean square of sucessive differences"), wobei aber auch andere Methoden, wie z.B. die Methode "SDRR" mit Hilfe von Standardabweichungen, die Methode pRR50 bei der die Anzahl der aufeinanderfolgenden RR-Intervalle ermittelt wird, die grösser als 50ms, und dieser Wert dividiert durch die Gesamtzahl aufeinanderfolgender RR-Intervalle oder Frequenzorientierte Methoden wie beispielsweise die Berechnung über die Quotienten $LF_{tot}/HF_{tot}$ der niederfrequentigen Frequenzanteile durch die höherfrequentigen Frequenzanteile. Der HRV Wert mittels RMSSD wird berechnet als die Quadratwurzel der Summe der quadrierten Differenzen zwischen benachbarten RR-Intervallen. In diesem Zusammenhang sollte darauf hingewiesen werden, dass bei der Wahl der Berechnungsmethode einerseits die jeweiligen neueren Erkenntnisse des jeweiligen Fachgebietes bzw. des jeweiligen Einsatzspektrums des erfindungsgemässen Verfahren bzw. der erfindungsgemässen Vorrichtung berücksichtigt werden können, aber andererseits auch rein praktische Gesichtspunkte der jeweiligen Auswahl denkbar sind. Im Falle der Ermittlung der HRV-Werte durch die RMSSD-Methode wird im hier vorliegenden Ausführungsbeispiel als Tabellenwert für $HRV_{min}$ für jedes Alter als 0 eingesetzt. Die im hier gezeigten Ausführungsbeispiel verwendeten anderen Minimalwerte für den Puls bzw. HRV werden gemäss der folgenden Tabelle gewählt:

Altersabhängige Ruhepulswerte

| Jugendlich: 14...18 | Ruhepuls: 85 Schläge/Minut |
|---|---|
| Erwachsene: 19...65 | Ruhepuls: 70 Schläge/Minute |
| Senioren: 65+ | Ruhepuls: 90 Schläge/Minute |

Altersabhängige $HRV_{max}$ Werte (RMSSD)

| 15...20 | 47ms |
|---|---|
| 21...30 | 46ms |
| 31...40 | 40ms |
| 41...50 | 35ms |
| 51...60 | 30ms |
| 61...70 | 24ms |

nach Angelink et al: Innovationstagung FH Rapperswil 4.5.2011

Es sollte dabei beachtet werden, dass - ohne den Sinn des erfindungsgemässen Verfahren zu verlassen, durchaus auch andere Parameter zu den Probanden, wie z.B. das Geschlecht etc. in die Tabelle einfliessen können.

Gemäss dem Ausführungsbeispiel wird die Normierung jeweils mittels eines Normierungswertes

$$P_z = P_{min} + a*(P_{max} - P_{min})$$

$$HRV_z = HRV_{min} + b* (HRV_{max} - HRV_{min})$$

und die Berechnung der Summanden für des Stresswert SI jeweils mittels

$$SI_P = (P_{d1} - P_z)/(P_{max} - P_z) \text{ wenn } P_{d1} > P_z$$

$$SI_P = (P_{d1} - P_z)/(P_z - P_{min}) \text{ wenn } P_{d1} < P_z$$

$$SI_{HRV} = -(HRV_{d1} - HRV_z)/(HRV_{max} - HRV_z) \text{ wenn } HRV_{d1} > HRV_z$$

$$SI_{HRV}=-(HRV_{d1}-HRV_z)/(HRV_z-HRV_{min}) \text{ wenn } HRV_{d1} < HRV_z$$

bzw.

$$SI_P=(P_{dx}-P_z)/(P_{max}-P_z) \text{ wenn } P_{dx} > P_z$$

$$SI_P=(P_{dx}-P_z)/(P_z-P_{min}) \text{ wenn } P_{dx} < P_z$$

$$SI_{HRV}=-(HRV_{dx}-HRV_z)/(HRV_{max}-HRV_z) \text{ wenn } HRV_{dx} > HRV_z$$

$$SI_{HRV}=-(HRV_{dx}-HRV_z)/(HRV_z-HRV_{min}) \text{ wenn } HRV_{dx} < HRV_z$$

durchgeführt, wobei a gewählt wurde als 0.25 und b als 0.5 und c und d gewählt als 1. Die aktuellen Werte des Stressindex SI nach der vorgegebenen Anzahl von Pulsschlägen gewählt werden im Ausführungsbeispiel mittels eines digitalen Tiefpassfilter $SI = f*SI_x+(1-f)*SI_{x-1}$ berechnet mit f von 0.1. Die Vorrichtung wurde entsprechend eingerichtet.

Es sollte noch bemerkt werden, dass die einzelnen Fenster, in denen die Stressindexwerte ermittelt werden, vorteilhafterweise für diverse Zustände des Probanden einschliessen, wie z.B. liegend, stehend, sich - im Sinne des Conconitest - bewegend etc.

Weiterhin sollte darauf hingewiesen werden, dass selbst bei zeitlich sehr weit auseinanderliegenden Testintervallen die Übernahme des letzten Testintervalls zu einem besseren bzw. zu einem schneller guten Ergebnis führt als wenn man mit den Tabellenwerten starten muss. Andererseits kann es selbstverständlich angezeigt sein, wieder Tabellenwerte zu benutzen, wenn sich der Zustand des Probanden grundsätzlich erheblich geändert hat.

## Patentansprüche

1. Verfahren zum Erkennen und Melden eines Belastungszustandes einer Person, wobei das Verfahren folgende Schritte umfasst:

   ● fortlaufendes Erfassen der Daten der momentanen Pulsfrequenz P und der momentanen Herzratenvariabilität HRV,
   ● fortlaufendes Verarbeiten der Daten der momentanen Pulsfrequenz P und der momentanen Herzratenvariabilität HRV,
   ● Vergleichen der gewonnenen momentanen Zustandsfunktion der Person mit einem Alarmkriterium,
   **dadurch gekennzeichnet, dass**
   in einem ersten Zeitintervall $T_1$ oder einer vorgegebenen Anzahl von Pulsschlägen ein Wert für den Stressindex SI bestimmt wird durch die Addition eines Wertes $SI_P$ für den Stressindex gewonnen aus einem normierten Durchschnittswert $P_{d1}$ des Pulses im genannten ersten Zeitintervall $T_1$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten ersten Zeitintervall $T_1$ oder über die vorgegebene Anzahl von Pulsschlägen

$$SI_1= c* SI_P + d* SI_{HRV}$$

   wobei die Normierung mittels Tabellenwerten $P_{max}$, $P_{min}$, $HRV_{max}$ und $HRV_{min}$ durchgeführt wird mittels aus altersabhängigen Minimal- und Maximal Puls und HRV-Werten, sowie die Maximal- und Minimalwerte der gemessenen Pulswerte und der HRV-Werte im Zeitintervall $T_1$ oder während der vorgegebenen Anzahl von Pulsschlägen bestimmt werden, wobei $T_1$ zwischen 100s und 1000 s, vorzugsweise mit 300s, oder die vorgegebene Anzahl von Pulsschlägen zwischen 50 und 500 liegt und vorzugsweise 100 ist, in zumindest einem weiteren Zeitintervall $T_x$(x= 2...n) oder während einer weiteren vorgegebenen Anzahl von Pulsschlägen der Stressindex wiederum bestimmt wird durch die Addition eines Wertes $SI_P$ für den Stressindex gewonnen aus einem normierten Durchschnittswert $P_{d1}$ des Pulses im genannten Zeitintervall $T_x$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten Zeitintervall $T_x$ oder über die vorgegebene Anzahl von Pulsschlägen

$$SI_x = c* SI_P + d* SI_{HRV}$$

vorzugsweise mit gleicher Länge wie $T_1$ oder bei gleicher Anzahl von Pulsschlägen,
wobei die Normierung mittels Werten $P_{max}$, $P_{min}$, $HRV_{max}$ und $HRV_{min}$ durchgeführt wird, wobei $P_{max}$ bzw. $HRV_{max}$ gewählt werden aus dem grösseren Wert von $P_{max}$ bzw $HRV_{max}$ bestimmt im vorherigen Zeitintervall $T_{x-1}$, oder während der vorherigen, vorgegebenen Anzahl von Pulsschlägen und dem vorherigen Zeitintervall $T_{x-1}$ oder während der vorgegebenen Anzahl von Pulsschlägen verwendeten Wert von $P_{max}$ bzw $HRV_{max}$, $P_{min}$ bzw. $HRV_{min}$ gewählt werden aus dem kleineren Wert von $P_{min}$ bzw. $HRV_{min}$ aus dem vorherigen Zeitintervall $T_{x-1}$ oder während der vorherigen, vorgegebenen Anzahl von Pulsschlägen und dem vorherigen Zeitintervall $T_{x-1}$ oder während der vorherigen, vorgegebenen Anzahl von Pulsschlägen verwendeten Wert von $P_{min}$ bzw. $HRV_{min}$.

2. Verfahren zum Erkennen und Melden eines Belastungszustandes einer Person nach Anspruch 1, wobei die Normierung jeweils mittels eines Normierungswertes

$$P_z = P_{min} + a*(P_{max} - P_{min})$$

$$HRV_z = HRV_{min} + b* (HRV_{max} - HRV_{min})$$

und die Berechnung der Summanden für des Stresswert SI jeweils mittels

$$SI_P = (P_{d1} - P_z)/(P_{max} - P_z) \text{ wenn } P_{d1} > P_z$$

$$SI_P = (P_{d1} - P_z)/(P_z - P_{min}) \text{ wenn } P_{d1} < P_z$$

$$SI_{HRV} = -(HRV_{d1} - HRV_z)/(HRV_{max} - HRV_z) \text{ wenn } HRV_{d1} > HRV_z$$

$$SI_{HRV} = -(HRV_{d1} - HRV_z)/(HRV_z - HRV_{min}) \text{ wenn } HRV_{d1} < HRV_z$$

bzw.

$$SI_P = (P_{dx} - P_z)/(P_{max} - P_z) \text{ wenn } P_{dx} > P_z$$

$$SI_P = (P_{dx} - P_z)/(P_z - P_{min}) \text{ wenn } P_{dx} < P_z$$

$$SI_{HRV} = -(HRV_{dx} - HRV_z)/(HRV_{max} - HRV_z) \text{ wenn } HRV_{dx} > HRV_z$$

$$SI_{HRV} = -(HRV_{dx} - HRV_z)/(HRV_z - HRV_{min}) \text{ wenn } HRV_{dx} < HRV_z$$

durchgeführt wird.

3.  Verfahren nach Anspruch 2, wobei a gewählt wird als 0.25 und b als 0.5.

4.  Verfahren nach einem der Ansprüche 1 bis 3, wobei c und d gewählt werden als 1.

5.  Verfahren nach einem der Ansprüche 1 bis 4, wobei der aktuelle Stressindex SI nach dem Zeitintervall $T_x$ oder nach der vorgegebenen Anzahl von Pulsschlägen gewählt wird mittels eines digitalen Tiefpassfilter SI = $f*SI_x+(1-f)*SI_{x-1}$ mit f zwischen 0.05 und 0.5, vorzugsweise von 0.1.

6.  Verfahren nach einem der Ansprüche 1 bis 5, wobei sich die Zeitintervalle bzw. die Zeiten, in denen eine vorgegebene Anzahl von Pulsschlägen gemessen wird, überlappen.

7.  Verfahren nach einem der Ansprüche 1 bis 5, wobei die Zeitintervalle bzw. die Zeiten, in denen eine vorgegebene Anzahl von Pulsschlägen gemessen wird, untereinander einen festen oder variablen Abstand haben.

8.  Vorrichtung zum Erkennen und Melden eines Belastungszustandes einer Person, mit:

    ● einer Erfassungseinrichtung zum fortlaufenden Erfassen der Daten der momentanen Pulsfrequenz und der momentanen Herzratenvariabilität,
    ● einer Verarbeitungseinrichtung zum fortlaufenden Verarbeiten der Daten der momentanen Pulsfrequenz und der momentanen Herzratenvariabilität, und
    ● einer Vergleichseinrichtung zum Vergleichen der gewonnenen momentanen Zustandsfunktion der Person mit einem Alarmkriterium, wobei
    **dadurch gekennzeichnet, dass**
    die Verarbeitungseinrichtung so eingerichtet ist, dass in einem ersten Zeitintervall $T_1$ oder einer vorgegebenen Anzahl von Pulsschlägen ein Wert für den Stressindex SI bestimmt wird durch die Addition eines Wertes $SI_P$ für den Stressindex gewonnen aus einem normierten Durchschnittswert $P_{d1}$ des Pulses im genannten ersten Zeitintervall $T_1$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten ersten Zeitintervall $T_1$ oder über die vorgegebene Anzahl von Pulsschlägen

$$SI_1 = c* SI_P + d* SI_{HRV}$$

wobei die Normierung mittels Tabellenwerten $P_{max}$, $P_{min}$, $HRV_{max}$ und $HRV_{min}$ durchgeführt wird mittels aus altersabhängigen Minimal- und Maximal Puls und HRV-Werten, sowie die Maximal- und Minimalwerte der gemessenen Pulswerte und der HRV-Werte im Zeitintervall $T_1$ oder während der vorgegebenen Anzahl von Pulsschlägen bestimmt werden, wobei $T_1$ zwischen 100s und 1000 s, vorzugsweise mit 300s, oder die vorgegebene Anzahl von Pulsschlägen zwischen 50 und 500 liegt und vorzugsweise 100 ist,
in zumindest einem weiteren Zeitintervall $T_x$(x= 2...n) oder während einer weiteren vorgegebenen Anzahl von Pulsschlägen der Stressindex wiederum bestimmt wird durch die Addition eines Wertes $SI_P$ für den Stressindex gewonnen aus einem normierten Durchschnittswert $P_{d1}$ des Pulses im genannten Zeitintervall $T_x$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten Zeitintervall $T_x$ oder über die vorgegebene Anzahl von Pulsschlägen

$$SI_x = c* SI_P + d* SI_{HRV}$$

vorzugsweise mit gleicher Länge wie $T_1$ oder bei gleicher Anzahl von Pulsschlägen,
wobei die Normierung mittels Werten $P_{max}$, $P_{min}$, $HRV_{max}$ und $HRV_{min}$ durchgeführt wird, wobei $P_{max}$ bzw. $HRV_{max}$ gewählt werden aus dem grösseren Wert von $P_{max}$ bzw $HRV_{max}$ bestimmt im vorherigen Zeitintervall $T_{x-1}$, oder während der vorherigen, vorgegebenen Anzahl von Pulsschlägen und dem vorherigen Zeitintervall $T_{x-1}$ oder während der vorgegebenen Anzahl von Pulsschlägen verwendeten Wert von $P_{max}$ bzw $HRV_{max}$, $P_{min}$ bzw. $HRV_{min}$ gewählt werden aus dem kleineren Wert von $P_{min}$ bzw. $HRV_{min}$ aus dem vorherigen Zeitintervall $T_{x-1}$ oder während der vorherigen, vorgegebenen Anzahl von Pulsschlägen und dem vorherigen Zeitintervall $T_{x-1}$ oder während der vorherigen, vorgegebenen Anzahl von Pulsschlägen verwendeten Wert von $P_{min}$ bzw. $HRV_{min}$.

9. Vorrichtung nach Anspruch 8, wobei die Vorrichtung so eingerichtet ist, dass das Verfahren gemäss Anspruch 2 durchgeführt werden kann.

10. Vorrichtung nach Anspruch 8 oder 9, wobei a gewählt wird als 0.25 und b als 0.5 und /oder wobei c und d gewählt werden als 1 und/oder der aktuelle Stressindex SI nach dem Zeitintervall $T_x$ oder nach der vorgegebenen Anzahl von Pulsschlägen gewählt wird mittels eines digitalen Tiefpassfilter

$$SI = f*SI_x + (1-f)*SI_{x-1}$$

mit f zwischen 0.05 und 0.5, vorzugsweise von 0.1.

Europäisches
Patentamt

European
Patent Office

Office européen
des brevets

**EUROPÄISCHER RECHERCHENBERICHT**

Nummer der Anmeldung

EP 12 18 8701

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 316 333 A1 (EIDGENOESSISCHE MATERIALPRUEFUNGS UND FORSCHUNGSANSTALT EMPA [CH]) 4. Mai 2011 (2011-05-04)<br>* Seite 2, Zeile 39 - Zeile 56 *<br>* Absatz [0008] *<br>* Absatz [0010] *<br>* Absatz [0023] *<br>* Seite 3, Zeile 15 *<br>* Seite 4, Zeile 54 *<br>* Seite 5, Zeile 10 *<br>* Seite 5, Zeile 18 - Zeile 50 *<br>* Seite 7, Zeile 31 *<br>* Seite 8, Zeile 33 - Zeile 36 *<br>----- | 1-10 | INV.<br>A61B5/024<br>A61B5/0402 |
| A | WO 00/51677 A2 (CHILDRE DOC L [US]; MCCRATY ROLLIN I [US]; ATKINSON MICHAEL A [US]) 8. September 2000 (2000-09-08)<br>* Seite 19, Zeile 24 - Zeile 26 *<br>* Seite 22, Zeile 10 - Zeile 19 *<br>----- | 1-10 | |
| A | WO 2007/123923 A2 (MIROW SUSAN [US])<br>1. November 2007 (2007-11-01)<br>* Seite 50, Absatz 000187 *<br>* Ansprüche 29, 36 *<br>----- | 1-10 | RECHERCHIERTE SACHGEBIETE (IPC)<br><br>A61B<br>G06F |
| A | WO 2007/057032 A1 (ENERGY LAB TECHNOLOGIES GMBH [DE]; SCHWARZ GEROLD [DE]; WEITL MARC [DE]) 24. Mai 2007 (2007-05-24)<br>* Seite 2, Zeile 20 - Zeile 22 *<br>* Seite 9, Zeile 14 *<br>* Seite 11, Zeile 8 - Zeile 10 *<br>----- | 1-10 | |
| A,D | DE 103 19 361 A1 (BIOSIGN GMBH [DE])<br>2. Dezember 2004 (2004-12-02)<br>* Absatz [0020] *<br>----- | 1-10 | |

-/--

Der vorliegende Recherchenbericht wurde für alle Patentansprüche erstellt

| Recherchenort | Abschlußdatum der Recherche | Prüfer |
|---|---|---|
| Den Haag | 28. März 2013 | Spirrov, Dimitar |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

**EP 2 721 999 A1**

<table>
<tr><td colspan="4" align="center">**EUROPÄISCHER RECHERCHENBERICHT**</td><td align="right">**Nummer der Anmeldung**<br>EP 12 18 8701</td></tr>
</table>

Europäisches Patentamt
European Patent Office
Office européen des brevets

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2011/137235 A1 (CYBERONICS INC [US]; OSORIO IVAN [US]; FREI MARK [US]) 3. November 2011 (2011-11-03) * Seite 23, Zeile 12 - Zeile 24 * ----- | 1-10 | |
| A | US R E38 749 E1 (DARDIK IRVING I [US]) 28. Juni 2005 (2005-06-28) * Abbildung 2 * * Spalte 7, Zeile 26 - Spalte 8, Zeile 10 * ----- | 1-10 | |
| A | US 2007/056582 A1 (WOOD MICHAEL [US] ET AL) 15. März 2007 (2007-03-15) * Abbildungen 3, 56, 58 * * Absatz [0230] * ----- | 1-10 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 28. März 2013 | Spirrov, Dimitar |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**          EP 12 18 8701

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-03-2013

| Im Recherchenbericht angeführtes Patentdokument | | Datum der Veröffentlichung | Mitglied(er) der Patentfamilie | | Datum der Veröffentlichung |
|---|---|---|---|---|---|
| EP 2316333 | A1 | 04-05-2011 | KEINE | | |
| WO 0051677 | A2 | 08-09-2000 | AT | 349240 T | 15-01-2007 |
| | | | AU | 770323 B2 | 19-02-2004 |
| | | | AU | 3507200 A | 21-09-2000 |
| | | | CA | 2364032 A1 | 08-09-2000 |
| | | | CN | 1358074 A | 10-07-2002 |
| | | | DE | 60032581 T2 | 04-10-2007 |
| | | | DK | 1156851 T3 | 02-04-2007 |
| | | | EP | 1156851 A2 | 28-11-2001 |
| | | | ES | 2282097 T3 | 16-10-2007 |
| | | | HK | 1042056 A1 | 20-07-2007 |
| | | | JP | 4410234 B2 | 03-02-2010 |
| | | | JP | 2002537911 A | 12-11-2002 |
| | | | JP | 2007083065 A | 05-04-2007 |
| | | | PT | 1156851 E | 30-03-2007 |
| | | | US | 6358201 B1 | 19-03-2002 |
| | | | US | 2007021675 A1 | 25-01-2007 |
| | | | US | 2009137915 A1 | 28-05-2009 |
| | | | WO | 0051677 A2 | 08-09-2000 |
| WO 2007123923 | A2 | 01-11-2007 | EP | 2007277 A2 | 31-12-2008 |
| | | | US | 2009292180 A1 | 26-11-2009 |
| | | | WO | 2007123923 A2 | 01-11-2007 |
| WO 2007057032 | A1 | 24-05-2007 | KEINE | | |
| DE 10319361 | A1 | 02-12-2004 | KEINE | | |
| WO 2011137235 | A1 | 03-11-2011 | AU | 2011245315 A1 | 01-11-2012 |
| | | | CA | 2797268 A1 | 03-11-2011 |
| | | | EP | 2563210 A1 | 06-03-2013 |
| | | | WO | 2011137235 A1 | 03-11-2011 |
| US RE38749 | E1 | 28-06-2005 | KEINE | | |
| US 2007056582 | A1 | 15-03-2007 | US | 2007056582 A1 | 15-03-2007 |
| | | | US | 2011313263 A1 | 22-12-2011 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 10319361 A1
- DE 10006154 A1
- DE 102006039957 A1
- DE 102008030956 A1
- EP 1156851 B1
- EP 2136333 A1